# EUROPEAN PATENT APPLICATION

(11) **EP 0 722 744 A1**
(43) Date of publication of application: **24.07.1996**
(21) Application number: 96300332.2
(22) Date of filing: 17.01.1996
(51) Int. Cl.: A61M 1/34

(54) **Apparatus for continous blood purification**

(30) Priority: 19.01.1995 JP 6185/95; 29.06.1995 JP 163479/95
(71) Applicant: UBE INDUSTRIES, LTD., Ube-shi, Yamaguchi-ken 755 (JP)
(72) Inventor: Tanaka, Masaaki, Chiba-city, Chiba-pref. (JP); Matsuno, Masao, Ichihara-city, Chiba-pref. (JP)
(74) Representative: Jackson, Peter Arthur

(57) **Abstract**

An apparatus for continuous blood purification having a function of simultaneous hemodialysis and hemofiltration includes: a blood circuit outside the body (an extracorporeal blood circuit) between a vein or an artery and a vein is provided with a blood purifier (a hemodiafilter); a circuit for introducing dialysate contained in a container into the blood purifier; a dialysate pump for transmitting the dialysate to the circuit for introducing dialysate; a circuit for introducing replacement fluid contained in a container connected to an extracorporeal blood circuit between a blood purifier and vein where blood return to the body so as to replace body fluid after hemofiltration; a replacement-fluid pump for transmitting the replacement fluid to the circuit for introducing replacement fluid; a circuit for discharging waste fluid obtained after blood purification (hemodiafiltration); and a waste-fluid pump in the circuit for discharging waste fluid; wherein each of the three pumps functions independently, each has a means for intermittently measuring a volume of dialysate, replacement fluid, or waste fluid, respectively, and a rotational frequency of each pump can be adjusted so that the obtained value coincide with a preset value.

## Description

The present invention relates to an apparatus for continuous blood purification. In the apparatus, blood is introduced from a vein or an artery into a blood purifier such as a hemofilter, where permeable substances such as water, electrolytes, and metabolites are filtered and separated from blood. Then, therapeutic utilities are supplemented so as to recover a patient's self-homeostasis, thereby supporting or substituting functions of internal organs.

Such various methods for purifying blood as whole-blood exchange, plasma exchange, hemoadsorption, hemodialysis, hemofiltration, hemodiafiltration, peritoneal dialysis, or the like have been widely applied to clinical medicine.

There have been practically performed to treat patients who suffer from renal failure by methods of blood purification for a long period of time in the field of clinical medicine. Renal failure is caused by its original organ's failure, or other sickness, or overhydration as a result of overinfusion of drugs solution during or after surgery.

In a hemofiltration, a water containing physiological substances passes through a membrane having pores from a blood side to the other side by the trans-membrane pressure which is derived from the difference of pressures between a pressure at a side and that at the opposite side of membrane. Physiological substances which passes through a pore is limited by the size or molecular weight of the physiological substances and the size of the pore. This enables selective removal by a principle of ultrafiltration. In this case, components of body fluid in the water removed are usually supplemented with supplementary fluid (replacement fluid).

In a treatment given to a patient having an acute or chronic renal failure, there is employed an intermittent hemodialysis or an intermittent hemodiafiltration, either of which usually completes the treatment for 4 - 5 hours. A volume of dialysate used for one time of treatment is about 120 litter, and the flow rate is about 500 ml/min. Further, about 20 litter of water is filtered besides a filtration of water corresponding to a weight increase of the patient (usually 1.5 - 2 litter) during the 4 - 5 hours, and instead, about a replacement fluid of about 20 litter is infused.

However, when this treatment is given to a serious patient in a short period of time, there is a case that a body fluid gets out of balance suddenly leading to a sudden turn for the worse of pathophysiological condition of the patient (imbalance syndrome). Therefore, a method for gradual treating a patient, i.e., a cure of continuous blood purification is rapidly spreading as an effective cure for a serious patient. This therapeutic manner is performed to a patient mainly in rooms for intensive care such as ICU, CCU, or the like, under a strict control of a body fluid balance. A certain institution has reported that a method for continuous blood purification took 1 hour for the shortest treatment, 2302 hours for the longest treatment, and 180.7 hours for the average treatment. As this report indicates, the continuous blood purification therapy has to be performed gradually with spending a long time, thereby requiring more strict control of body fluid balance of patients. Therefore, a highly precise apparatus exceeding a conventional common sense has recently been required.

There has conventionally been used a roller pump, which transmits fluid inside of an elastic soft tube by drawing outside of an elastic soft tube, as an apparatus (a pump) for transmitting fluid for blood purification (hemocatharsis).

However, a tube often used for a roller pump is usually made of plastics such as poly (vinyl chloride), thereby having variance in a tube diameter based on fluctuant manufacturing condition of tube or thereby changing in a cross-sectional area because of deformation of a tube due to a pressure change inside a tube during transmitting fluid through it. Therefore, an obtained value does not coincide with a preset value of the pump. Generally, errors are within the range from about plus or minus 5% to about plus or minus 15%. A degree of an error depends on a circuit. Particularly, a filtrate pump is most liable to be influenced by a change of a pressure. That is, in the course of treating, platelets, protein in blood, and the like, adhere to a blood purifier, and a filtration resistance gradually increases. When the filtrate pump rotates with a constant rotational frequency, a pressure in a filtrate side of a blood purifier decreases according to an increase of a filtration resistance. The decrease of a pressure causes a deformation (decrease of a cross-sectional area) of a tube, thereby decreasing a flow rate. This is well known in the scene of cure. Practically, filtrate is received by a measuring container such as a measuring cylinder and measured the volume point by point so as to adjust a rotational frequency of a filtrate pump to keep the flow rate constant.

To measure a volume of filtrate by a measuring cylinder is not only hard but also imprecise. Moreover, it sometimes happens that a person in charge forgets measuring the volume, thereby causing overflow cf filtrate from a measuring cylinder.

As described above, controlling a flow rate of filtrate by a conventional method for continuous blood purification is so hard that a caretaker is required to stay with a patient throughout the treatment to avoid a sudden change in patient's condition which is brought by a change in flow rate. Thus, a conventional method cannot use its merit to the full. Besides, a conventional method is in danger of making a patient serious on the contrary. Therefore, there has been expected an appearance of an apparatus which can automatically adjust a value of an actual flow rate so as to be close to a preset value with high reliability.

The object of the present invention is to provide an apparatus for continuous blood purification which can automatically and precisely measure and control an amount of filtrate so as to maintain the flow rate of filtrate constantly and precisely in a continuous blood purification treatment, particularly a continuous hemofiltration as described above.

In the first aspect of the present invention, there is provided an apparatus for continuous blood purification, comprising: a blood purifier into which blood is introduced from a vein or an artery of a patient, in which the introduced blood is purified, and from which the purified blood is returned to a vein; a blood-outgoing circuit which introduces blood from a vein or an artery into the blood purifier; a blood pump in the blood-outgoing circuit; a filtrate circuit through which a filtrate from the blood purifier is discharged; a filtrate pump in the filtrate circuit; and a means for intermittently measuring a volume of filtrate flow, installed in the filtrate circuit in the outlet side of the filtrate pump; wherein a rotational frequency of the filtrate pump is adjusted so that the obtained value of the filtrate coincide with a preset value.

The means for intermittently measuring a volume of filtrate flow comprises: a plastic measuring chamber connected to the apparatus by being branched off the filtrate circuit in the outlet side of the filtrate pump; and a tube clamp positioned in a tubed flow path extending from a branch portion of the filtrate circuit to the discharge end of the tube.

Preferably, the blood pump and the filtrate pump have transporting capacities of 15 - 250 ml/min. and 50 - 2,000 ml/hr., respectively. Further, errors of the filtrate pump in measuring an integrated flow amount are preferably within plus or minus 1%. More preferably, the content volume of a plastic measuring chamber is 10 - 30 ml.

Furthermore, the measuring chamber is preferably provided with an opening-shutting means so as to control ventilation in the upper portion of the measuring chamber, and the opening-shutting means is shut so as to stop an inflow of air into the measuring chamber after the filtrate is discharged from the chamber.

In another aspect of the invention, there is provided an apparatus for continuous blood purification, comprising: a blood purifier into which blood is introduced from a vein or an artery of a patient, in which the introduced blood is purified, and from which the purified blood is returned to a vein, the blood purifier having a function of a simultaneous hemodialysis and hemofiltration; a blood circuit outside the body (an extracorporeal blood circuit) positioned between a vein (an artery) and a vein; a circuit for introducing dialysate contained in a container into the blood purifier; a dialysate pump for transmitting the dialysate to the circuit for introducing dialysate; a circuit for introducing replacement fluid contained in a container connected to an extracorporeal blood circuit between a purifier and vein where blood returns to a body so as to replace body fluid components after hemofiltration; a replacement-fluid pump for transmitting the replacement fluid to the circuit for introducing replacement fluid; a circuit for discharging waste fluid obtained after blood purification (hemodiafiltration); and a waste-fluid pump in the circuit for discharging waste fluid; wherein each of the three pumps functions independently, each has a means for intermittently measuring a volume of dialysate, replacement fluid, or waste fluid, respectively, and a rotational frequency of each pump can be adjusted so that the obtained value coincide with a set value.

Preferably, transporting capacities of the dialysate pump, the replacement-fluid pump, and the discharge pump are 100 - 2,000 ml/hr, 50 - 1,000 ml/hr, and 75 - 3,000 ml/hr, respectively, and errors of each of the dialysate pump, the replacement-fluid pump, and the waste-fluid pump in measuring an integrated flow amount are within plus or minus 1%.

Incidentally, a means for measuring filtrate of the present apparatus has a measuring chamber having a certain capacity and calculates a volume of filtrate flow precisely by measuring a volume of filtrate in the chamber at regular intervals. When the actual flow rate is lower than a preset flow rate by a pump, a rotational frequency of the pump is increased, and when the actual flow rate is higher than a preset flow rate by a pump, a rotational frequency of the pump is decreased, thereby adjusting an actual flow rate so as to become close to the preset flow rate. Errors of a conventional roller pump are within the range from about plus or minus 5% to about plus or minus 15%, while errors is within plus or minus 1% after ten hours' operation by adjusting a flow rate by the present method. By employing the present controlling mechanism, there can be provided an apparatus having high precision which exceeds common sense as an apparatus for continuous hemodiafiltration.

In the accompanying drawings:

Fig. 1 is a flowchart showing an embodiment of an apparatus for continuous blood purification of the present invention.

Figs. 2A, 2B, and 2C are schematic views showing a function of an opening-shutting valve for controlling ventilation in the upper tube connected to a measuring container.

Fig. 3 is a graph showing the results of Table 1. The axis of ordinates shows a filtrate flow rate, and the axis of abscissas shows time course. The closed circle (●) means that a measuring chamber was not used, and the opened circle (○) means that a measuring chamber was used after 2 hours from the start.

Fig. 4 shows a relation between time course in the axis of abscissas and errors of the filtrate pump in measuring an integrated flow amount in the axis of ordinates on the basis of the results of Fig. 3.

Fig. 5 is a flowchart showing another embodiment of an apparatus for continuous blood purification of the present invention.

The present invention is hereinbelow described with reference to the embodiments in the figures. However, the present invention is by no means limited to the embodiments.

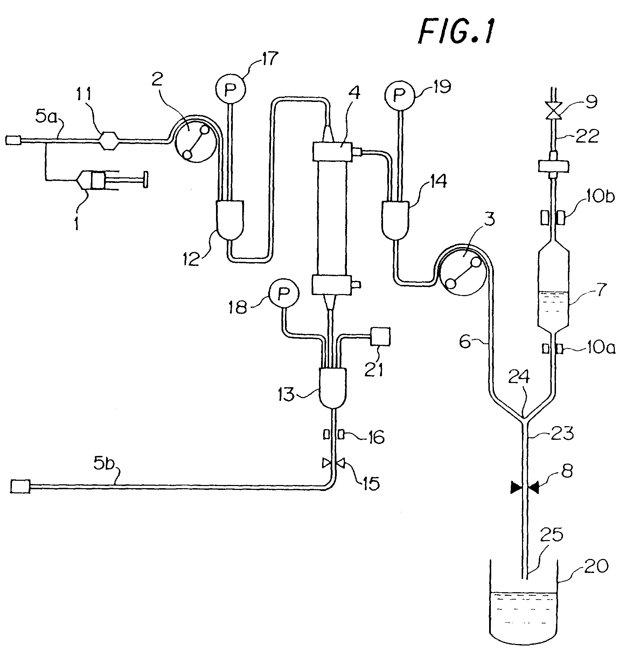

Fig. 1 is a flowchart showing an embodiment of an apparatus for continuous blood purification of the present invention.

In the Fig. 1, a flow rate of blood to be taken out and a flow rate of an anticoagulant to be injected are determined depending on a patient's condition. The anticoagulant is injected with a syringe pump 1. Blood is taken out of patients and introduced into the blood circuit 5a and 5b including a blood purifier 4 by a blood pump 2. A flow rate of filtrate to be obtained by filtering with a blood purifier 4 is also determined depending on a patient's condition, and a value of a flow rate of a filtrate pump 3 is set. Incidentally, transmitting capacities of the blood pump 2 and the filtrate pump 3 are preferably 15 - 250 ml/min. and 50 - 2,000 ml/hr., respectively.

Blood taken out of a patient with a blood pump 2 is introduced into a blood purifier 4 through a blood-outgoing circuit 5a. The blood is filtered to remove waste matters and purified by a blood purifier 4 and returned to the patient through a blood-returning circuit 5b. In the blood purifier 4, solute containing waste matters is ultrafiltered by the difference of pressures generated between a side and the opposite side of membrane (trans-membrane pressure) by a rotation of a filtrate pump 3, thereby purifying blood.

Filtrate obtained by filtration with a blood purifier 4 is discharged into a waste-fluid container 20 through a filtrate circuit 6.

In Fig. 1, 11 denotes a pillow for detecting a decrease of blood volume from a patient. Each of 12, 13, and 14 denotes a pressure chamber for measuring a pressure in a circuit. 15 denotes a clamp for air bubbles protection, 16 denotes a sensor for air bubbles. Each of 17, 18, and 19 denotes a pressure sensor. 21 denotes a portion for supplying replacement fluid.

Measurement of a volume of filtrate flow is now described.

When a filtrate pump 3 is rotating, an opening-shutting valve 9 is open, and a filtrate clamp 8 is shut; a filtrate flows into a measuring chamber 7. When the surface of the filtrate (boundary between filtrate and air) passes through a lower filtrate-level sensor 10a and further reaches an upper filtrate-level sensor 10b, the filtrate clamp 8 is opened with the opening-shutting valve 9 remaining open, thereby the filtrate in the measuring chamber 7 flows out of the chamber 7 by the difference of height (a head). After a predetermined period of time, the filtrate clamp 8 is shut, and filtrate flows into a measuring chamber 7 again. When the surface of the filtrate passes through the lower filtrate-level sensor 10a and further reaches the upper filtrate-level sensor 10b, the filtrate clamp 8 is opened and the filtrate in the measuring chamber flows out of the chamber. This cycle is repeated by a predetermined period of time. The time T required for filtrate to flow into a measuring chamber is measured as a time until the surface of the filtrate reaches the upper filtrate-level sensor 10b after the surface passes the lower filtrate-level sensor 10a. Because the measuring chamber 7 is produced with using a plastic molding die, the production hardly causes errors. Therefore, the measuring chamber 7 has a fixed capacity of V₀. A rate L₀ of filtrate flow passing through a filtrate pump 3 is: L₀ = V₀/T₀.

When a value of the rate Lo is lower than a preset value, a rotational frequency of the filtrate pump 3 is increased, and when a value of the rate L₀ is higher than a set value, a rotational frequency of the filtrate pump 3 is decreased, thereby controlling the rate L₀ so as to be close to the preset value.

Though the predetermined period of time depends on a rate of filtrate flow, the time is preferably set by 1 - 20 minutes so as to improve the preciseness. Preferably, the time is set to be longer when a flow volume is small, and the time is set shorter when a flow volume is large.

An upper tube 22 connected to the measuring chamber 7 is set to the opening-shutting valve 9. After filtrate is discharged and the surface of the filtrate passes the lower filtrate-level sensor 10a, the opening-shutting valve 9 is closed so that flow of air into the chamber 7 and an excessive fall of the surface of the filtrate in a discharge tube 23 be stopped. If the upper tube 22 is still open after the filtrate in the chamber 7 is discharged, air is sucked into the tube 23 from the side of the measuring chamber 7 by a negative pressure, thereby generating an air bubble. After starting measurement, the air bubble passes through the lower filtrate-level sensor 10a, affects a detection of a proper surface, and causes errors in measuring.

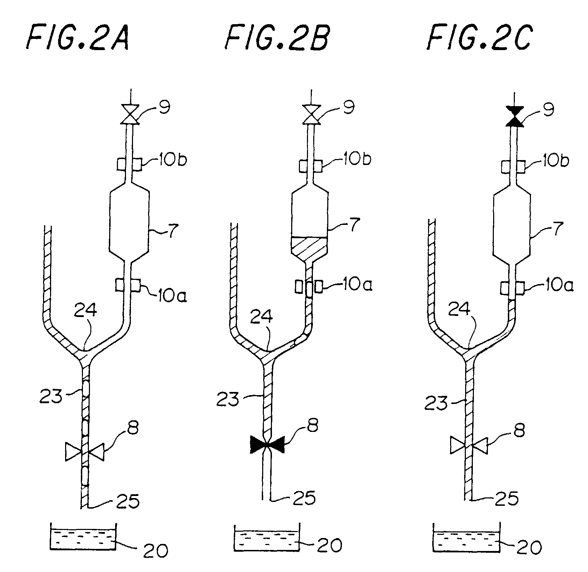

This point is described in more detail on the basis of Figs. 2A, 2B, and 2C. If the apparatus is not provided with the opening-shutting valve 9, or if the opening-shutting valve 9 is kept open, air is sucked in from the side of the measuring chamber 7, as shown in Fig. 2A, because of a negative pressure caused by a head (difference of height) between a bifurcation 24 and an end 25 of the tube 23 toward the waste-fluid container 20.

If the clamp 8 is shut with keeping this condition and the measuring of filtrate is started, an air bubble enters the tube in the side of the measuring chamber 7, thereby the surface of filtrate passes the lower filtrate-level sensor 10a a plurality of times which causes difficulties to distinguish an exact surface to start measuring. To avoid this situation in the apparatus of the present invention, the lower filtrate-level sensor 10a constantly observes a surface of the filtrate, and if another surface of the filtrate is detected before the upper filtrate-level sensor 10b detects the surface of the filtrate during measuring, the measuring is cancelled. In the case, the filtrate is once discharged, and the measurement is started again. If such a condition is repeated many times, the measurement becomes impossible practically.

If an air bubble is mixed in filtrate, the filtrate sometimes cannot be smoothly discharged. That is, when the end 25 of a tube 23 is deeply immersed into the filtrate collected in a waste-fluid container 20, a head is balanced with an buoyant force of the air bubble, causing oppilation in the tube 23, and the filtrate sometimes reaches the measuring chamber 7. In such a case, measurement may become impossible practically because the surface of the filtrate should be lower than the lower filtrate-level sensor 10a when the measurement is started.

In this embodiment, the filtrate in the measuring chamber 7 is discharged after the measuring is finished. The aforementioned mixing of an air bubble is avoided by shutting the opening-shutting valve 9 with a slight time lag after the surface of the filtrate passes through the lower filtrate-level sensor 10a as shown in Fig. 2C.

The opening-shutting valve 9 fixed to the upper tube 22 connected to the measuring chamber 7 is preferably electromagnetic from the view points of convenience in operation and avoidance of omission in the control.

A filtrate-level sensor is preferably ultrasonic sensor which is not influenced by a color of filtrate because filtrate is a waste matter of body fluid and colored.

Incidentally, a blood purifier gradually increases a filtration resistance because of gradual adhesion of platelets, protein in blood, and the like. If a rotational frequency of a filtrate pump is fixed, a volume of filtrate flow decreases as described above. However, the increase of a filtering resistance is usually slow. Therefore, even if long intervals are taken in measuring, usually, a change of flow rate during each interval can be ignored and a big error does not happen.

However, it is more preferable to start measuring soon after a measured filtrate is completely discharged because it means the filtrate amount is measured all the time except for the inconsiderable time to be spent for discharging filtrate in the measuring chamber, and therefore, even if the volume of filtrate flow is unexpectedly changed, a high precision of flow rate is realized.

An interval of measuring time can be easily set by using a known means such as a timer. A control of filtrate flow with higher precision, e.g., errors of the filtrate pump in measuring an integrated flow amount being within plus or minus 1%, can be realized by setting the interval of measurement very short, thereby measuring almost continuously.

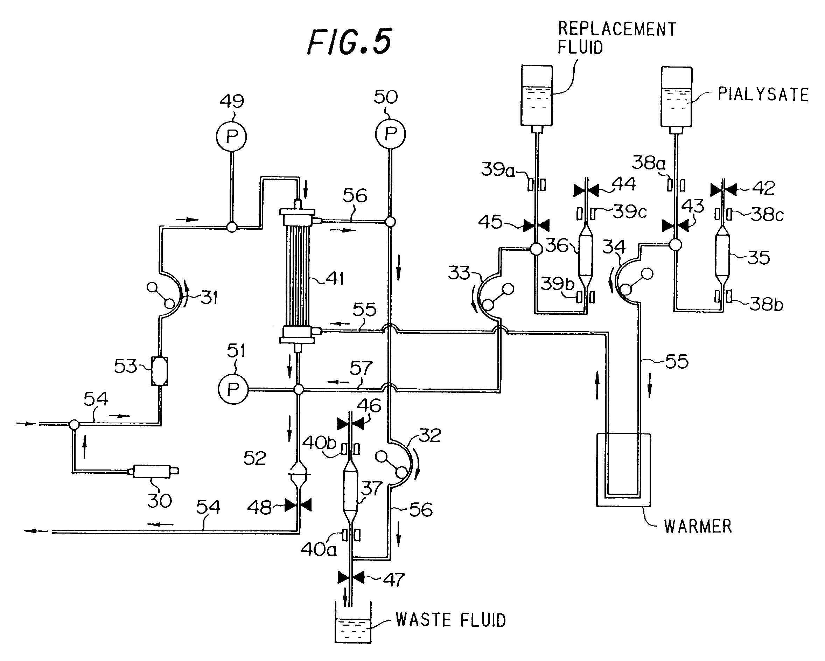

Fig. 5 is a flowchart showing another embodiment of an apparatus for continuous blood purification of the present invention.

In Fig. 5, a flow rate of blood to be taken out and a flow rate of an anticoagulant to be injected are determined depending on a patient's condition. The anticoagulant is injected with a syringe pump 30. Blood is taken out of patients and introduced into blood circuit 54 including a blood purifier 41 by a blood pump 31. A flow rate of dialysate and a flow rate of filtrate to be obtained by filtering with a blood purifier 41 is also determined depending on a patient's condition, and a value of a flow rate of a dialysate pump 34 and a value of a flow rate of a waste-fluid pump 32 are set.

A flow rate of waste fluid coincides with a total flow rate of dialysate and filtrate.

Since values of flow rates of dialysate, waste fluid, and replacement fluid are precisely controlled just as preset; each pump, each level sensor, and each clamp operate as follows. Incidentally, transmitting capacities of a dialysate pump, a replacement-fluid pump, and a waste-fluid pump are desirably 100 - 2,000 ml/hr., 50 - 1,000 ml/hr., and 75 - 3,000 ml/hr., respectively.

Measurement of a flow volume of dialysate is now described.

A dialysate pump 34 is rotated, and clamps 42 and 43 are opened. When dialysate is transmitted to a circuit 55 for dialysate, dialysate simultaneously flows into the measuring chamber 35 because of a head between the surface of the dialysate in a container for dialysate and a surface of dialysate in the measuring chamber 35. When the surface of dialysate reaches an upper level sensor 38c for dialysate, the clamp 42 is opened, and the clamp 43 is shut. The dialysate in a measuring chamber flows out of the measuring chamber and is transmitted to the side of the dialysate pump. When the surface of the dialysate passes the lower level sensor 38b for dialysate, the clamp 42 is shut, the clamp 43 is opened, and transmission of dialysate is continued. If the clamp 42 is opened after a predetermined period of time, dialysate again flows into the chamber. When the surface of dialysate passes the upper level sensor 38c for dialysate, a clamp 42 is opened and a clamp 43 is closed and dialysate flows out of the measuring chamber. This cycle is repeated by a predetermined period of time. The time T₁ required for dialysate to flow out of the measuring chamber is measured as a time until the surface of dialysate passes a lower level sensor 38b after the surface of the dialysate passes the upper level sensor 38c for dialysate. Since the measuring chamber 35 for dialysate is produced with using a plastic molding die, the production hardly causes errors. Therefore, the measuring chamber 35 has a fixed capacity of V₁. A flow rate L₁ of dialysate passing through a pump 34 is: L₁ = V₁/T₁.

When the flow rate L₁ is lower than a preset value, a rotational frequency of the pump 34 is increased, and when the flow rate L₁ is higher than a preset rate, a rotational frequency of the pump 34 is decreased, thereby controlling the rate L₁ so as to be closer to the preset value, i.e., to make errors of the pump in measuring an integrated flow amount within plus or minus 1%.

When a predetermined period of time is short and measuring is conducted soon after the dialysate finishes flowing out, the operation can be repeated without shutting the clamp 42.

Measurement of a volume of waste-fluid flow is now described.

When a waste-fluid pump 32 is rotated, the clamp 46 is opened, and the clamp 47 is shut; waste fluid flows into a measuring container 37 for waste fluid. When the surface of the waste fluid reaches an upper level sensor 40b for waste fluid after passing through a lower level sensor 40a for waste fluid; the clamps 46 and 47 are opened, and the waste fluid in a measuring chamber 37 for waste fluid flows out of the chamber by a head. After a predetermined period of time, the clamp 47 is shut and waste fluid again flows into the measuring chamber 37. When the surface of the waste fluid reaches the upper level sensor 40b for waste fluid after passing the lower level sensor 40a for waste fluid; the clamp 47 is opened, and waste fluid in the measuring chamber flows out of the chamber. This cycle is repeated by a predetermined period of time. The time T₂ required for waste fluid to flow in the measuring chamber is measured as a time until the surface of waste fluid reaches the upper level sensor 40b for waste fluid after the surface of the waste fluid passes the lower level sensor 40a for waste fluid. Since the measuring chamber 37 is produced with using a plastic molding die, the production hardly causes errors. Therefore, the measuring chamber 37 has a fixed capacity of V₂. A rate L₂ of waste fluid passing through a waste-fluid pump 32 is: L₂ = V₂/T₂.

When a value of the rate L₂ is lower than a preset value, a rotational frequency of the pump 32 is increased, and when a value of the rate L₂ is higher than a preset value, a rotational frequency of the pump 32 is decreased, thereby controlling the rate L₂ so as to be closer to the preset value, i.e., to make errors of the pump in measuring an integrated flow amount within plus or minus 1%.

Measuring of a volume of replacement-fluid flow is now described.

A replacement-fluid pump 33 is rotated, and the clamps 44 and 45 are opened. When replacement fluid is transmitted to a replacement-fluid circuit 57 by a pump; simultaneously, the replacement fluid flows into a measuring chamber 36 for replacement fluid by a head between the surface of replacement fluid in a chamber and the surface of replacement fluid in a measuring chamber 36 for replacement fluid. When the surface of the replacement fluid reaches the upper level sensor 39c for replacement fluid, the clamp 44 is opened, and the clamp 45 is shut. The replacement fluid flows out of the measuring chamber for replacement fluid and is transmitted to the replacement-fluid pump 33. When the surface of the replacement fluid passes the lower level sensor 39b for replacement fluid, the clamp 45 is opened, the clamp 44 is shut, and transmission of the replacement fluid is continued. After a predetermined period of time, the clamps 44 and 45 are opened, and replacement fluid again flows into the measuring chamber 36. When the surface of replacement fluid reaches the upper level sensor 39c for replacement fluid, the clamp 44 is opened, the clamp 45 is shut, and replacement fluid in the measuring chamber flows out of the chamber. This cycle is repeated by a predetermined period of time. The time T₃ required for replacement fluid to flow out of the measuring chamber 36 is measured as a time until the surface of replacement fluid passes the lower level sensor 39b for replacement fluid after the surface of the replacement fluid passes the upper level sensor 39c for replacement fluid. Since the measuring chamber 36 is produced with using a plastic molding die, the production hardly causes errors. Therefore, the measuring chamber 36 has a fixed capacity of V₃. A rate L₃ of replacement fluid passing through the pump 33 is: L₃ = V₃/T₃.

When a value of the rate L₃ is lower than a preset value, a rotational frequency of the pump 33 is increased, and when a value of the rate L₃ is higher than a preset value, a rotational frequency of the pump 33 is decreased, thereby controlling the rate L₃ so as to be closer to the preset value, i.e., to make errors of the pump in measuring an integrated flow amount within plus or minus 1%.

A predetermined period of time is short, and when measuring is conducted soon after the replacement fluid finishes flowing out, the operation can be repeated without shutting the clamp 44.

Though the predetermined period of time depends on a rate of replacement-fluid flow, the time is preferably set by 1 - 20 minutes so as to improve the preciseness. Preferably, the time is set to be longer when a flowing volume is small, and the time is set shorter when a flowing volume is large.

A conventionally known blood purifier (hemodiafilter) can be used for the present invention, and a hollow fiber membrane module which uses ultrafiltration membrane of hollow fibers as a filtering material is most suitable.

Specifically, a blood purifier has about 5000 - 20000 of hollow fibers installed in a plastic case. The plastic case is made of polycarbonate, polysulfone, or the like. The hollow fiber is made of polyacrylonitrile, polysulfone, polyamid, cellulose acetate, polyethylene, poly(vinyl alcohol), polypropylene, poly methylmethacrylate, ethylene vinyl alcohol, or the like.

A hollow fiber suitable for hemodiafiltration is made of cellulose acetate, polyacrylonitrile, polysulfone, poly methylmethacrylate, polyamid, ethylene vinyl alcohol, or the like.

Generally, a hollow fiber has an inner diameter of about 100 - 600 µm and a thickness of a membrane is about 50 - 150 µm. A diameter of a pore formed in a hollow fiber is selected depending on the object such as hemodialysis, hemofiltration, hemodiafiltration, plasma separation. A hollow fiber membrane module having an surface area of a membrane of 0.1 - 1.0 m² is preferably used, and a hollow fiber membrane module having a suitable surface area of a membrane is selected depending on the condition of a patient.

When blood is purified, there are two methods. One method is that blood introduced into a hollow fiber is filtered toward outside the hollow fiber. The other method is that dialysate is introduced in the periphery of a hollow fiber simultaneously with the introduction of blood into the hollow fiber, and substances are transferred by a dialytic phenomenon simultaneously with a physical separation of substances in dialysate from inside the hollow fiber.

A blood circuit to be used for an apparatus of the present invention is constituted by conventionally known members such as a plastic tube, a drip chamber, a pressure-detecting port portion, a pressure-monitoring pillow, a tube connector, and the like. These members are made of a resin such as poly (vinyl chloride), polyacrylonitrile, polyolefin such as polypropylene, polycarbonate, poly (methyl methacrylate), and polysulfone. Among these materials, poly vinyl chloride is preferable in consideration of a transforming ability particularly for tube.

A measuring chamber to be used for an apparatus of the present invention is preferably made of a hard resin for the object of measurement, and specifically, poly (vinyl chloride), poly (methyl methacrylate), polyacrylonitrile, polycarbonate, polypropyrene, polysulfone, or the like, can be used. Incidentally, though a measuring chamber does not need to be transparent in view of measuring function, the measuring chamber is preferably transparent so as to watch and confirm a normal operation of measurement. The measuring chamber preferably has an prismal or a cylindrical shape.

A kind of pump for the present invention is not limited, and various kinds of pumps such as a roller-pump, a finger pump (peristaltic type pump), or the like can be used. Each of these pumps has inherent error in ability on measurement, and the nominal value set according to the scale of the pump differs from the observed real value. However, an apparatus of the present invention is provided with a means for measuring a real volume, and a rotational frequency of each pump can be adjusted so that an observed value by measuring coincide with a preset value. Therefore, in the present invention, there can be used a roller pump, which is generally thought to have large errors.

### ( Embodiment 1 )

In the apparatus of the present invention shown in Fig. 1, a value of a filtrate flow rate was fixed to be 500 ml/hr., and a predetermined period of time was three minutes. A fluid (service water) in a closed container was sucked and discharged by a filtrate pump 3 under the controlling of a rotational frequency of the pump. The integrated flow amount of the discharged fluid (service water) during 2.5 hours was measured by an electronic scale. The weight was 1247g and the error was -0.24%.

Incidentally, a pressure in the closed container was reduced from an ambient pressure before the start of suction to -303mmHg after 2.5 hours.

Further, fluid (for example, service water) was sucked and discharged by a filtrate pump 3 with fixing a flow rate in the filtrate pump 3 to be 500 ml/hr. without using a measuring chamber 7, i.e., without controlling a rotational frequency of the filtrate pump 3 depending on a result of measurement so as to compare with an effect of an apparatus of the present invention shown in Fig. 1. The integrated flow amount of the discharged fluid (service water) during 2.5 hours was measured by an electronic scale. The weight was 895g and the error was -28.4%.

Incidentally, a pressure in the closed container was reduced from an ambient pressure before the start of suction to -251mmHg after 2.5 hours.

As described above, according to an apparatus of the present invention, an influence of a pressure is avoided and an expected flow rate can be maintained even though a pressure in a tube is decreased.

In the apparatus shown in Fig. 1, relation between a rotational frequency of a pump and a flow rate is memorized in a computer installed in the system so as to control flow rate to be the same as the preset value when a pump tube made of specified materials and having specified size is used.

Table 1 shows changes in flow rate and amount using a pump tube made of specified material but having a slightly larger diameter than a specified size. These data were obtained by values shown by an electronic scale with sucking and discharging water in a opened container in an ambient pressure positioned on the electronic scale with use or without use of measuring chamber. This brings data for the effect of the size of tube on the flow rate and amount without the fluctuant effect of filtering resistance caused by a pressure decrease in a tube.

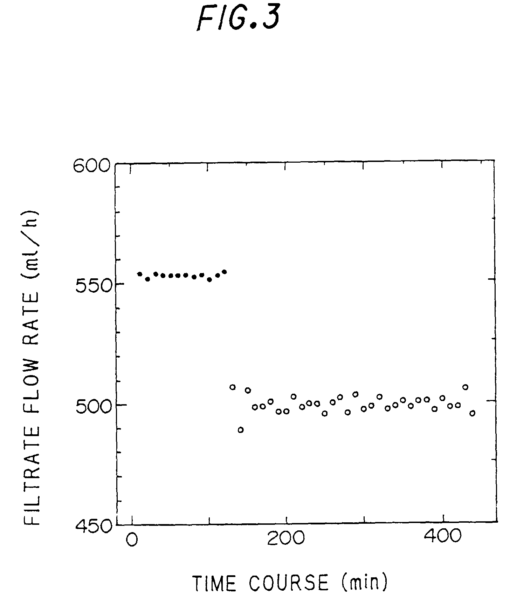

The data in Table 1 are illustrated graphically in Fig. 3. In this figure, closed circles (●) show flow rates through the filtrate pump 3, in which flow rate was preset at 500 ml/hr., without using a measuring chamber, i.e., a rotational frequency of the filtrate pump 3 was not controlled depending on a result of measuring. The opened circles (○) show rates through a filtrate pump 3 under a condition that measuring is started after 120 minutes and that a rotational frequency of the filtrate pump 3 was controlled on the basis of the results of measuring. A flow rate was calculated as differences of weighed values with an electronic scale at determined time. In the case of ○, soon after the measurement and the discharge of fluid in a measuring chamber, the next measurement was started. There was used a measuring chamber having a capacity of 20 ml.

As obvious from Fig. 3, an actual average flow rate obtained from the data shown by ● with using an electronic scale was 553.1 ml/hr., which is about 11% higher than a preset value of 500 ml/hr. because the size of a pump tube is slightly larger than a regular size. On the other hand, an average flow rate obtained from the data shown by ○, where the measurement and the control was conducted, was 499.4 ml/hr.

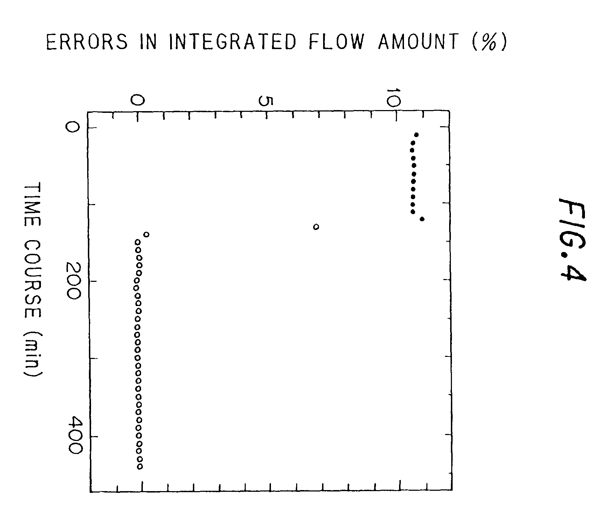

Fig. 4 shows errors of integrated flow amount on the basis of the results of Fig. 3. As obvious from Fig. 4, errors of ● were about 10.6%, while errors of ○, which was taken 320 minutes after the start of the measurement and the control, was -0.10%.

As described above, according to an apparatus of the present invention, a constant flow rate can be maintained even if an inner diameter of a pump tube is changed.

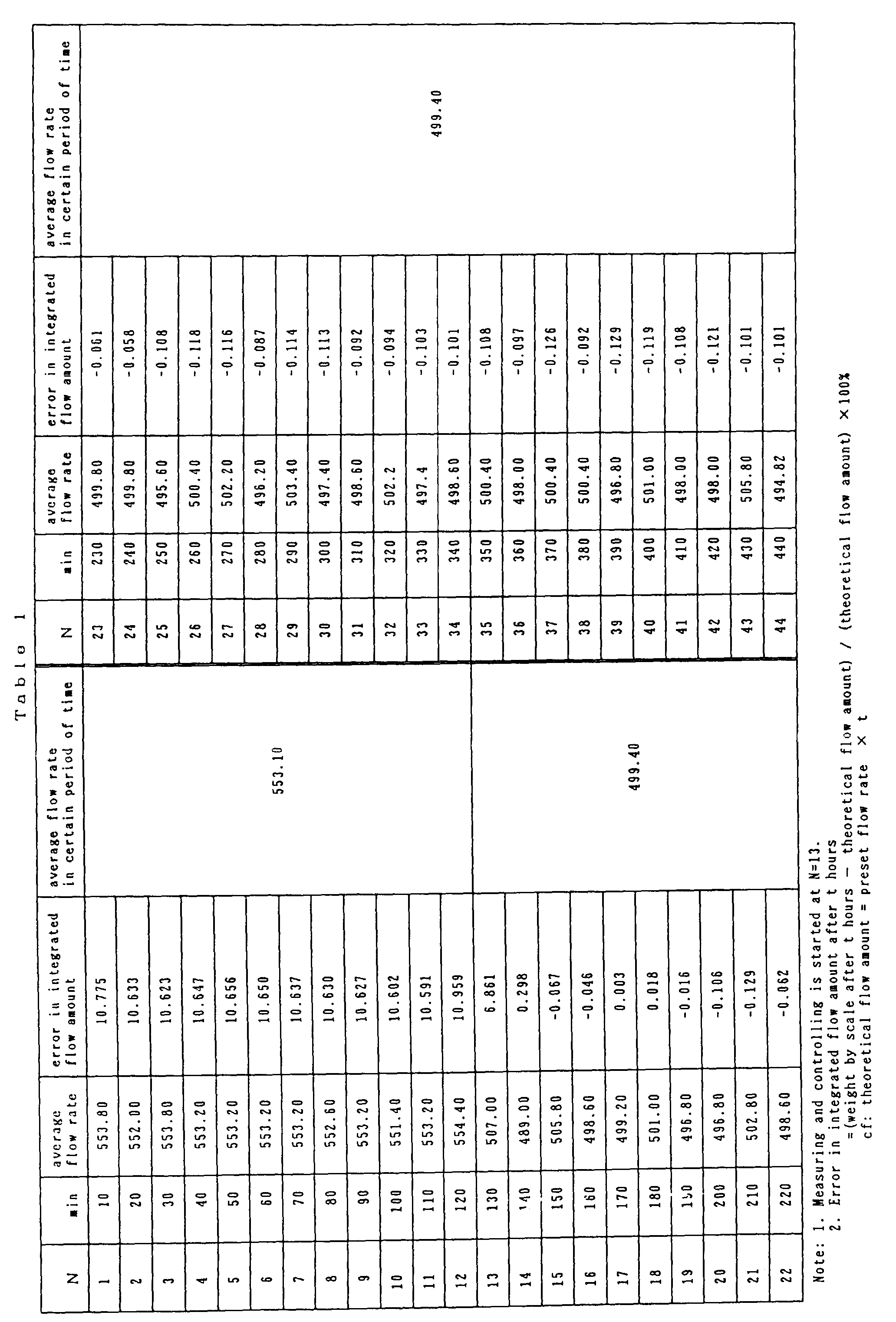

### ( Embodiment 2 )

In the apparatus of the present invention shown in Fig. 5, there were fixed a flow rate of dialysate through a pump to be 500 ml/hr. and a predetermined period of time to be every three minutes. An integrated flow amount was measured by an electronic scale when fluid (e.g., service water) was transmitted continuously for ten hours. The integrated flow amount was 5013 g, and the error is 0.26%.

In this system, a flow rate through a pump for waste fluid was fixed to be 500 ml/hr., and a predetermined time was fixed to be every three minutes. Fluid (service water) in a closed container was sucked and discharged by a waste-fluid pump under the controlling of a rotational frequency of the pump. An integrated flow amount of the discharged fluid (service water) for 3.5 hours was 1756 g, and the error was 0.34%.

Incidentally, a pressure in the closed container was reduced from an ambient pressure before starting the suction to -322mmHg after 3.5 hours.

Further, fluid (for example, service water) in a closed container was sucked and discharged by a filtrate pump 32 with fixing a flow rate in the filtrate pump 32 to be 500 ml/hr. without using a measuring chamber 37, i.e., without controlling a rotational frequency of the filtrate pump 32 depending on a result of measurement so as to compare with an effect of an apparatus of the present invention shown in Fig. 5. The integrated flow amount of the discharged fluid (service water) during 3.5 hours was measured by an electronic scale. The weight was 1433 g and the error was -18.1%.

Incidentally, a pressure in the closed container was reduced from an ambient pressure before the start of suction to -271mmHg after 3.5 hours.

As described above, in the present invention, a flow rate of a filtrate is intermittently measured by a means for measuring a volume, and a value obtained by the measuring is reflected in controlling a rotational frequency of a pump, thereby realizing a preset flow rate automatically with high precision. Therefore, an apparatus for continuous blood purifier of the present invention for a serious patient whose body fluid balance needs to be strictly controlled reduces a burden of a caretaker who is required controlling continuously body fluid balance of a patient. Further, there is no possibility of causing a serious problem which requires additional medical treatments because an apparatus of the present invention is highly precise.

## Claims

1. An apparatus for continuous blood purification, comprising:
a blood purifier into which blood is introduced from a vein or an artery of a patient, in which the introduced blood is purified, and from which the purified blood is returned to a vein;
a blood-outgoing circuit which introduces blood from a vein or an artery into the blood purifier;
a blood pump in the blood-outgoing circuit;
a filtrate circuit by which a filtrate from the blood purifier is discharged;
a filtrate pump in the filtrate circuit; and
a means for intermittently measuring a volume of filtrate flow, installed in the filtrate circuit in the outlet side of the filtrate pump;
wherein a rotational frequency of the filtrate pump is adjusted so that the obtained value of the filtrate coincide with a preset value.

2. An apparatus for continuous blood purification according to claim 1, wherein the means for intermittently measuring a volume of filtrate flow comprising:
a plastic measuring chamber connected to the apparatus by being branched off the filtrate circuit in the outlet side of the filtrate pump; and
a tube clamp positioned in a tubed flow path extending from a branch portion of the filtrate circuit to the discharge end of the tube.

3. An apparatus for continuous blood purification according to claim 2, wherein the measuring chamber is provided with an opening-shutting means so as to control ventilation in the upper portion of the measuring chamber, and the opening-shutting means is shut so as to stop an inflow of air into the measuring chamber after the filtrate is discharged from the chamber.

4. An apparatus for continuous blood purification according to claim 1, wherein the blood purifier is one of a hemodialyzer, a hemofilter, a hemoconcentrator, or a plasma separator.

5. An apparatus for continuous blood purification according to claim 1, wherein the measuring chamber comprises at least one resin selected from a group of poly (vinyl chloride), poly (methyl methacrylate), polyacrylonitrile, polycarbonate, polypropylene, and polysulfone.

6. An apparatus for continuous blood purification according to claim 1, wherein a tube constituting a circuit comprises at least one resin selected from a group of poly (vinyl chloride), polyolefin, polyacrylonitrile, polycarbonate, poly (methyl methacrylate), and polysulfone.

7. An apparatus for continuous blood purification according to claim 1, wherein the blood pump and the filtrate pump have transporting capacities of 15 - 250 ml/min. and 50 - 2,000 ml/hr., respectively.

8. An apparatus for continuous blood purification according to claim 1, wherein errors of the filtrate pump in measuring an integrated flow amount are within plus or minus 1%.

9. An apparatus for continuous blood purification according to claim 2, wherein the measuring chamber has a capacity of 10 - 30 ml.

10. An apparatus for continuous blood purification, comprising:
a blood purifier into which blood is introduced from a vein or an artery of a patient, in which the introduced blood is purified, and from which the purified blood is returned to a vein, the blood purifier having a function of a simultaneous hemodialysis and hemofiltration;
a blood circuit outside the body (an extracorporeal blood circuit) positioned between a vein or an artery and a vein;
a circuit for introducing dialysate contained in a container into the blood purifier;
a dialysate pump for transmitting the dialysate to the circuit for introducing dialysate;
a circuit for introducing replacement fluid contained in a container connected to an extracorporeal blood circuit between a blood purifier and vein where blood return to the body so as to replace body fluid after hemofiltration;
a replacement-fluid pump for transmitting the replacement fluid to the circuit for introducing replacement fluid;
a circuit for discharging waste fluid obtained after blood purification (hemodiafiltration); and
a waste-fluid pump in the circuit for discharging waste fluid;
wherein each of the three pumps functions independently, each has a means for intermittently measuring a volume of dialysate, replacement fluid, or waste fluid, respectively, and a rotational frequency of each pump can be adjusted so that the obtained value coincide with a preset value.

11. An apparatus for continuous blood purification according to claim 10, wherein transporting capacities of the dialysate pump, the replacement-fluid pump, and the waste-fluid pump are 100 - 2,000 ml/hr, 50 - 1,()00 ml/hr, and 75 - 3,000 ml/hr, respectively.

12. An apparatus for continuous blood purification according to claim 10 or 11, wherein errors of each of the pump for dialysate, the pump for replacement fluid, and the pump for waste fluid in measuring an integrated flow amount are within plus or minus 1% or less.
